# EUROPEAN PATENT SPECIFICATION

(11) **EP 2 117 439 B1**
(45) Date of publication and mention of the grant of the patent: **21.03.2012**
(21) Application number: 07702767.0
(22) Date of filing: 15.01.2007
(51) Int. Cl.: A61B 10/02, A61B 17/00, A61B 17/30

(54) **PNEUMATIC DEVICE FOR TAKING A TISSUE SAMPLE**
PNEUMATISCHE VORRICHTUNG ZUR ENTNAHME EINER GEWEBEPROBE
DISPOSITIF PNEUMATIQUE POUR PRÉLEVER UN ÉCHANTILLON TISSULAIRE

(43) Date of publication of application: 18.11.2009
(73) Proprietor: Janssens, Jacques Phillibert, 3500 Hasselt (BE)
(72) Inventor: Janssens, Jacques Phillibert, 3500 Hasselt (BE)
(74) Representative: Donné, Eddy
(86) International application number: PCT/EP2007/000299
(87) International publication number: WO 2008/086817

(56) References cited:
- EP-A- 1 676 532
- WO-A-98/33435
- WO-A-2007/051509
- US-A- 5 526 822
- US-A1- 2003 114 773
- US-A1- 2004 019 299
- US-A1- 2005 065 453
- US-A1- 2005 113 715

## Description

### BACKGROUND OF THE INVENTION.

### 1. Field of the invention.

This invention relates to a device for taking a tissue sample, in other words, to a biopsy device.

### 2. Discussion of the Related Art.

Devices for taking a tissue sample are already known in different forms.

Some types of such biopsy devices comprise several components, such as for example needles and/or canullas, which can be moved with respect to each other and which are driven by means of electric motors and driving gear, hereby providing automated movement.

A problem that is known to exist in the field of such biopsy devices is the magnetic and/or electromagnetic interference of the device with the so called MRI or Medical Resonance Imaging apparatuses.

### SUMMARY OF THE INVENTION

The invention aims at an improved device for taking a tissue sample, said device being of the type comprising at least two components which can be moved with respect to each other in a driven way, as opposed to the manually operable biopsy devices.

More particularly, the invention aims at an improved device offering the advantage that tissue samples can be taken very efficiently, safe for the patient, and without disturbing the eventual MRI apparatuses.

To this aim, the invention concerns a biopsy device according to claim 1. Biopsy devices with a needle assembly according to the preamble of claim 1 are known from US 2005/0113715 and from US 5.526.822. However these devices do not comprise a vacuum unit that is part of the needle assembly. Instead they use an external vacuum unit. The creation of a vacuum moreover is controlled by a separate control and needs to be coordinated with the movement of the components of the needle assembly.

A major advantage of the invention is the absence of electric motors and other electric power and/or switch devices that operate at high Voltage, which in normal operation create a magnetic field which can pose safety hazards for the devices, malfunctioning and disturb MRI apparatuses.

Another advantage that is presented in an unexpected way is that operation of the pneumatic devices is much more reliable and very suitable for operation in humid, sometimes bloody conditions.

Moreover, it is understood now that also the security for the patient is drastically improved thanks to the use of a biopsy device according to the invention.

Finally, since the action of the needles to harvest the tissue can be greatly improved by vacuum aspiration, air movements from and to the needles can be combined in one apparatus. The apparatus therefore can act both as a compressor and as an aspirator.

It is known that MRI apparatuses have evolved and are now capable to localise an injured tissue of only 1 mm in a very precise manner.

The potential that is offered by the accuracy of the present MRI apparatuses can now in full be exploited by using a device according to the invention.

According to a preferred embodiment, the needle assembly comprises components which are made of materials which are magnetically passive.

It is clear that various types of needle assemblies can be provided in co-operation with the pneumatic driving means.

A needle assembly can comprise for example a hollow piercing needle provided of a pointed distal end and immediately thereafter a tissue receiving port, for example in the form of an aperture in the jacket of the needle.

An outer cannular cutter can be provided over said hollow piercing needle, which cannular cutter fits precisely over the needle.

The outer cannular cutter is meant to be moved, i.e. to be translated while eventually being rotated, over said needle in order to cut of tissue which protrudes through the tissue receiving port.

Similar needle assemblies are provided of an inner cannular cutter instead of said outer cannular cutter.

Inside the hollow piercing needle, a tubular knock-out pin can be provided.

According to a preferred embodiment, the needle assembly comprises a spirally shaped tissue-receiving element and a cannular cutting element which can cooperate with the circumference of the spirally shaped tissue-receiving element in order to provide in a cutting function.

There is indeed an extremely advantageous and synergetic effect in providing such a needle assembly in combination with the main characteristics of a device according to the present invention, being the provision of a gas or liquid compressor and/or aspirator and conducting means for conducting compressed gas or liquid, for example air, from the compressor and/or aspirator to such a biopsy needle assembly comprising at least a spirally shaped tissue-receiving element and a cannular cutting element which can cooperate with the circumference of the spirally shaped tissue-receiving element in order to provide in a cutting function.

Indeed, the absence of electromagnetic driving means provides for a biopsy device that is extremely suitable for working in cooperation with an MRI apparatus, which allows very precise operation as explained above.

As opposed to the needle assemblies from which the tissue receiving element is a hollow piercing needle provided of a pointed distal end and immediately thereafter a tissue receiving port, for example in the form of an aperture in the jacket of the needle, a spirally shaped tissue-receiving element is provided of an aperture at the distal front end.

This feature of the aperture at the distal front end of the tissue receiving element allows direct or frontal approach of a suspect tissue.

An aperture in the jacket of a hollow needle requires lateral approach, pointing the needle up to a region aside the suspect tissue, which is known to be less accurate.

It is clear that the more accurate frontal approach of the suspect tissue results in an overall accurate biopsy if combined with precise Magnetic Resonance Imaging or other stereotactic imaging devices..

By preference, the outer free extremity of the spirally shaped tissue-receiving element extends in a direction which is situated in the prolongation of the spiral course of the spirally shaped tissue-receiving element.

The spirally shaped tissue-receiving element has as an advantage that it can easily be inserted into the tissue and subsequently a sample can be taken from the tissue in an efficient manner. At the same time, due to the spiral shape of the tissue-receiving element, a good anchoring between the tissue sample and the tissue-receiving element is made possible.

Due to the fact that the free extremity of the spirally shaped tissue-receiving element extends in the prolongation of the course of the spiral, during the screwing-in of the tissue-receiving element into the tissue, the portion of tissue which is located centrally in between the spiral, and which finally is to form the sample, is not touched by the spirally shaped tissue-receiving element and, as a consequence, is not damaged.

The fact that the free extremity extends in the prolongation of the spiral, also offers the advantage that the tissue-receiving element can be smoothly inserted in the tissue, only by a screwing force, without the need of creating an axial pushing force. Furthermore, due to the use of a cutting element which cooperates with the circumference of the spirally shaped tissue-receiving element, the portion of tissue caught in the spiral can be separated from the surrounding tissue in a very adequate and precise manner, with no or almost no damage done to the surrounding tissue of the patient.

Due to the sole axial movements of the needle parts the procedure can be done entirely in the anesthesia tract resulting in a pain free procedure.

Preferably, the tissue-receiving element has a helical course, as a consequence of which it can easily be brought into the tissue by means of a screw movement.

Further, the tissue-receiving element preferably shows one or more of the characteristics as disclosed in the claims of EP 1.359.848 B1, and/or as also disclosed in paragraphs 11 to 65 of the same document

The needle assembly and the transmission between the pneumatic driving means and the needle assembly can be carried out as disclosed in PCT/EP106/009179 .

### BRIEF DESCRIPTION OF THE DRAWINGS

With the intention of better showing the characteristics of the invention, hereafter, as an example without any limitative character, a preferred form of the embodiment is described, with reference to the accompanying drawings, wherein:
figure 1 schematically represents a device according to the invention;
figure 2 represents a schematic view of the part indicated with F2 in figure 1;
figure 3 represents a cross-section according to line III-III in figure 2;
figure 4 represents a cross-section according to line IV-IV in figure 3;
figures 5 to 7 represent a view and cross-sections of the part as indicated with F5 in figure 3;
figures 8 to 10 represent a view and cross-sections of the part as indicated with F8 in figure 3;
figures 11 to 13 represent a view and cross-sections of the part as indicated with F11 in figure 3;
figures 14 to 18 represent schematic views of the part indicated with F14 in figure 1, for different positions;
figure 19 represents schematic views of the part indicated with F19 in figure 1;
figure 20 represents a view according to arrow F20 in figure 19.

### DESCRIPTION OF THE PREFERRED EMBODIMENT.

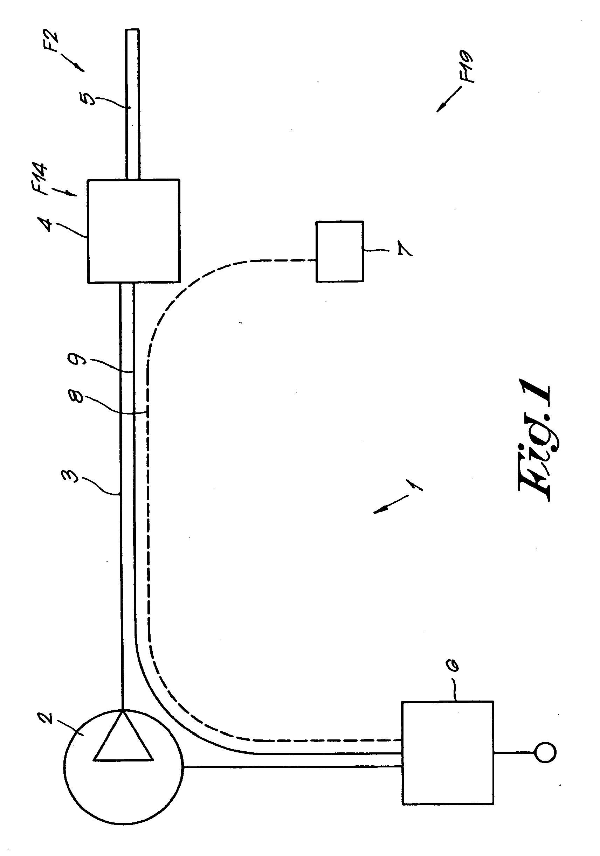

Figure 1 represents a preferred embodiment of a biopsy device 1 according to the invention.

The biopsy device 1 comprises a gas compressor 2 and conducting means 3, in this case three flexible tubes for conducting compressed gas, for example air, from the compressor 2 to a pneumatic driving means 4 which cooperates with a biopsy needle assembly 5.

The device 1 is additionally provided of a steering unit 6, in this case an electronic steering unit 6, which is provided of electric current and which is suitably connected with the compressor 2 for both providing electric power and for steering the compressor 2 in a desired manner.

The steering unit 6 is in this embodiment also connected with a user interface 7 provided near the needle assembly 5, in this case with a low voltage signal transmission cable 8 that follows the course of the conducting means 3 for conducting compressed gas between the compressor 2 and the pneumatic driving means 4.

The steering unit 6 is in this embodiment also connected with the pneumatic driving means 4, in this case with one or more low voltage signal transmission cables 9, that also follows the course of the conducting means 3 for conducting compressed gas between the compressor 2 and the pneumatic driving means 4. These cables 9 can be used for steering and transmitting feedback signals.

The biopsy needle assembly 5, is preferably as disclosed in PCT/EP106/009179 and, for reasons of clarity, partly resumed therefrom and discussed hereafter.

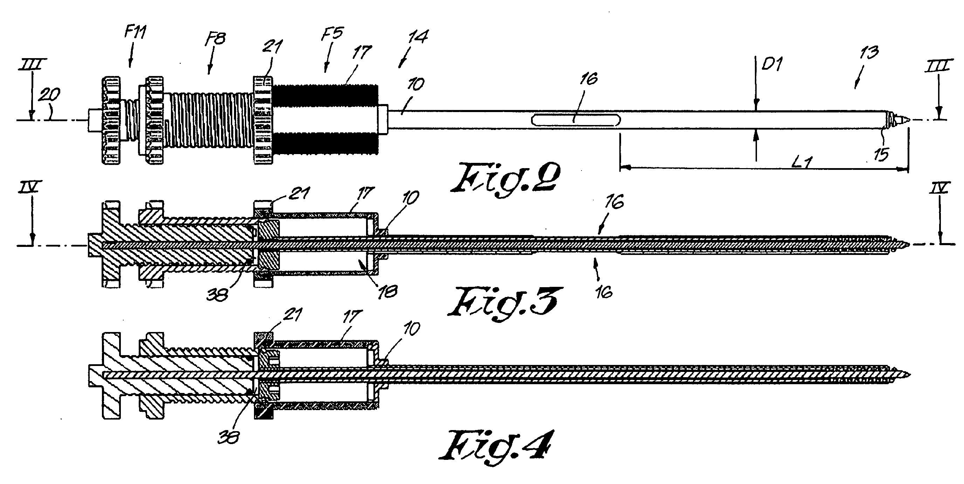

The biopsy needle assembly 5 mainly comprises three elements, a cannula 10, a hollow biopsy needle 11 and an inner needle 12, as represented in more detail in figures 2 to 4.

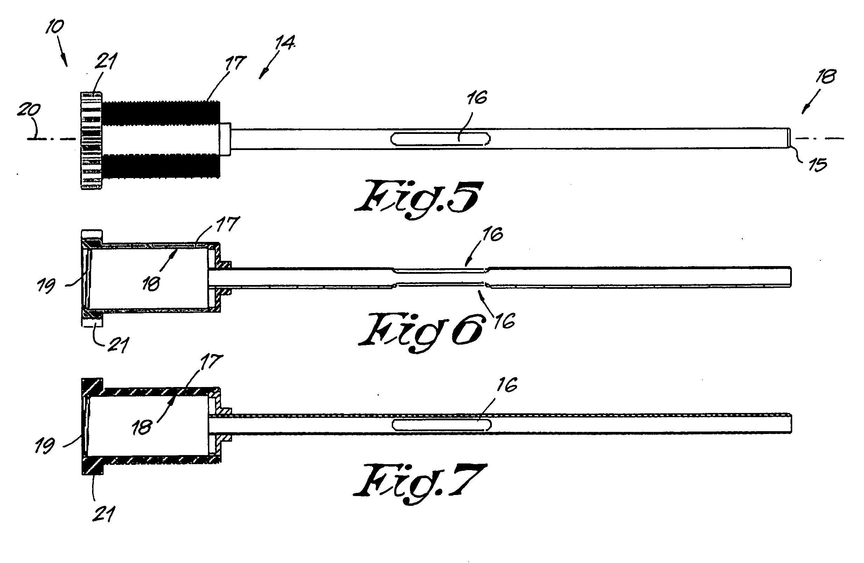

The cannula 10 is represented in more detail in figures 5 to 7, where it is indicated that the outer cannula 10 has a front end 13 and a back end 14, the front end 13 being for insertion in a tissue such as a patient tissue.

The front end 13 has a cutting edge 15 for cutting a tissue sample.

The cannula may comprise at least one, or more than one, aperture 16 for removal of the cut sample by the hollow biopsy needle 11.

The cannula 10 is provided with a cylindrical back end 17, whose inner side 18 comprises a screw thread 19. The cannula 10 is rotatably mounted around the axis 20 of the biopsy needle assembly 5. Its cylindrical back end 17 comprises a gear wheel 21 for engaging with a counter gear wheel for making the cannula rotate around the axis 20.

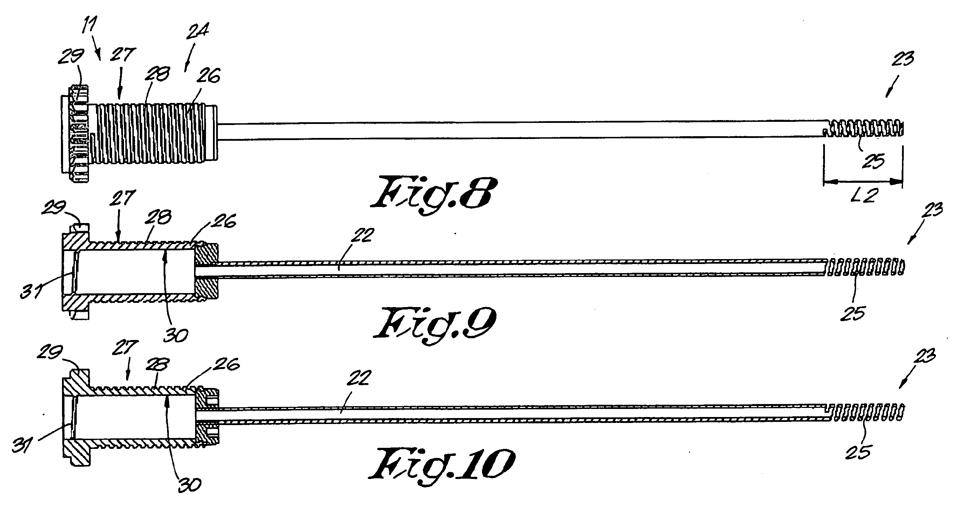

The hollow biopsy needle 11 is represented in more detail in figures 8 to 10. The hollow biopsy needle 11 is located inside and coaxially with the cannula 10. The hollow biopsy needle 11 has an inner cavity 22 and has a front end 23 and a back end 24. The front end 23 is provided with a tissue receiving means 25.

Preferably the tissue receiving means 25 is a spirally shaped tissue receiving means, such as disclosed in US 2003/0114773A1 . The hollow biopsy needle 11 has a cylindrical back end 26, and the cavity 22 of the hollow biopsy needle 11 extends into this cylindrical back end 26. Preferably, that hollow biopsy needle is rotatably mounted around the axis 20 of the biopsy needle assembly 5.

The outer surface 27 of the cylindrical back end 26 of the hollow biopsy needle 11 is provided with a screw thread 28 for engaging with the screw thread 19 of the inner side 18 of the cylindrical back end 26 of the cannula 10.

The cylindrical back end 26 of the hollow biopsy needle 11 is provided with a gear wheel 29 for engaging with a counter gear wheel in order to rotate the hollow biopsy needle 11 around the axis 20. At the inner side 30 of the cylindrical back end 26 of the hollow biopsy needle 11, a screw thread 31 is provided.

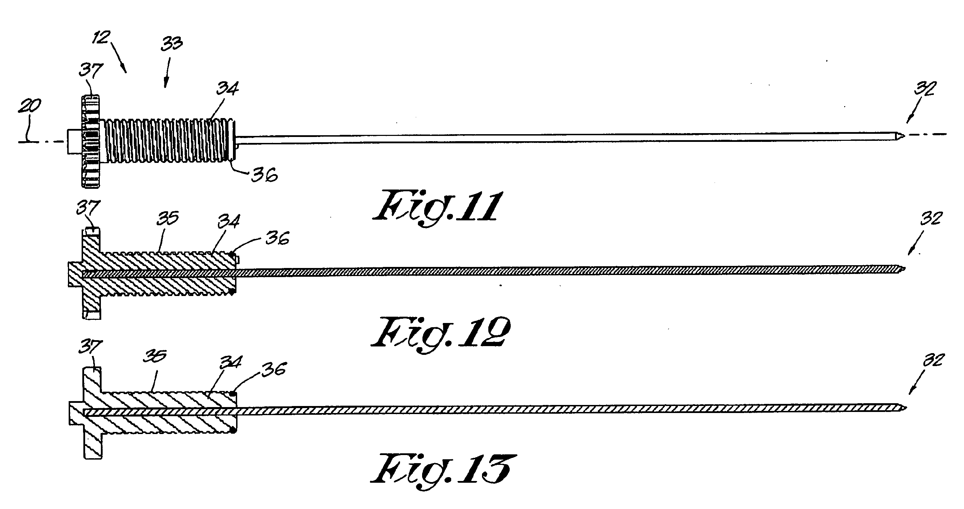

The biopsy needle assembly 5 comprises an inner tubular needle 12 or rod (trocar) which is represented in more detail in figures 11 to 13, having a front end 32 and a back end 33.

The inner needle 12 is located inside and coaxially with the hollow biopsy needle 11. The back end 33 of the inner needle 12 is provided with a cylindrical body 34, which is moveable inside the cylindrical back end 26 of the hollow biopsy needle 11.

The inner needle 12 is rotatably mounted around common axis 20 of cannula 10, hollow biopsy needle 11 and inner needle 12.

The cylindrical body 34 of the inner needle 12, together with the inner side 30 of the back end 24 of the hollow biopsy needle, make up a piston-cylinder vacuum unit for creating a lowered air pressure in said cavity 22 when said front end 23 of said hollow biopsy needle 11 is axially moved away from said front end 32 of said inner needle 12.

The cylindrical body 34 has a screw thread 35, for engaging with the screw thread 31 of said inner side 30 of said cylindrical back end 26 of said hollow biopsy needle 11.

The cylindrical body 34 comprises also means 36 to provide an airtight coupling of the cylindrical body 34 and cylindrical back end 26, e.g. a circular sealing member or joint. The cylindrical body 34 is provided with a gear wheel 37 for engaging with a counter gear wheel in order to rotate said inner needle around the axis 20.

According to the present invention, when the inner needle 12 is rotated relative to the hollow biopsy needle 11, the engagement of the screw thread 31 and screw thread 35 forces the inner needle 12 to move backwards from the hollow biopsy needle 11 or the hollow biopsy needle 11 is forced to move forward from the inner needle 12.

Thereby, the inner space 38 between the cylindrical body 34 and the cylindrical body 34 is increased in volume and the front end 23 of the hollow biopsy needle 11 is axially moved away from the front end 32 of the inner needle 12.

As there is an airtight coupling of the cylindrical body 34 and cylindrical back end 26, a vacuum or lowered air pressure is created in the inner space 38. This lowered air pressure is present in the inner cavity 22, as well as the cavity 22 extends into this inner space 38. This lowered air pressure, present in the inner cavity 22, creates suction or a vacuum at the tissue receiving means 25, more in particular at the location where the tissue receiving means 25 and outer end 32 of the inner needle 12 meet.

So, the cylindrical back end 26 and the cylindrical body 34 make up a piston-cylinder vacuum unit for creating a lowered pressure in the cavity 22 when the front end 23 of the hollow biopsy needle 11 is axially moved away from the front end 32 of the inner needle 12.

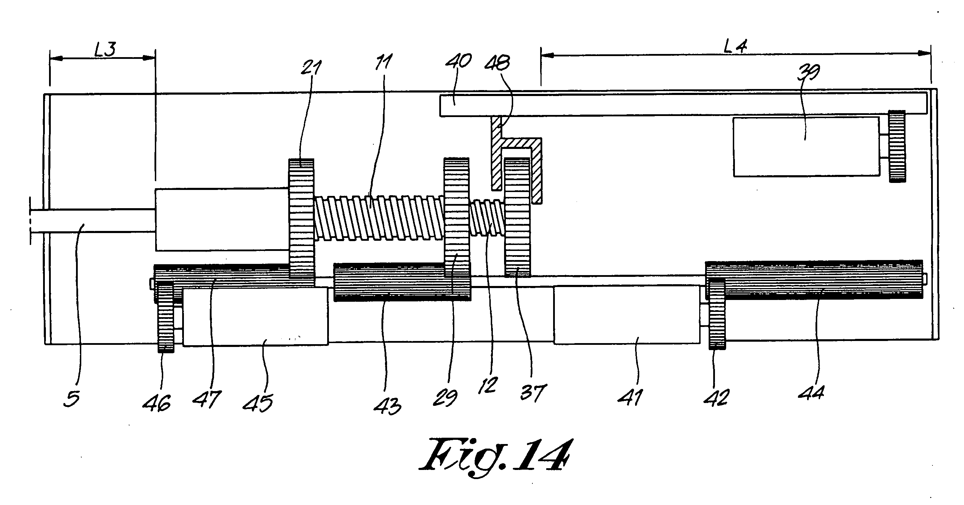

Figure 14 represents a pneumatic driving means 4 as indicated in figure 1, which co-operate with the biopsy needle assembly 5.

The pneumatic driving means 4 comprise means for moving said cutting canulla 10, hollow biopsy needle 11 and needle 12 axially forward and backward, which may be a pneumatic motor 39 by means of a set of gear wheels and a geared belt 40, may move the hollow biopsy needle 11 as well as motor 41, which drives a gear wheel 44 and 42, coupled.
to only the gearwheel 29 of the cylindrical back end 26 of the hollow biopsy needle 11 by means of a first counter gear wheel 43, or which may engage with the gearwheel 29 of the cylindrical back end 26 of the hollow biopsy needle 11 and the gear wheel 37 of the inner needle 12 by means of a second counter gear wheel 44.

These components, together with appropriate engaging or blocking of gear wheels, and appropriate co-operating of engaging screw threads, as will be explained below, causes the hollow biopsy needle 11 to move forward and backwards in an axial direction as well.

The gearwheels and by preference all or most parts of the pneumatic driving means 4 are made of plastics or magnetically passive materials, or the pneumatic driving means 4 are provided in a housing made of magnetic shielding materials.

The pneumatic driving means 4 comprises means for axially moving the cannula 10 forwards and backwards, these means comprising a pneumatic driving means, e.g. a pneumatic motor 45, which drives gear wheel 46 and counter gear wheel 47, the latter engaging with the gear wheel 21 of the cylindrical back end 17 of cannula 10.

Together with appropriate engaging or blocking of gear wheels, and appropriate cooperating of engaging screw treads, as will be explained below, causes the cannula 10 to move axially forwards and backwards.

The pneumatic driving means 4 comprises means for preventing the inner needle 12 to move in axial direction when the hollow biopsy needle 11 is axially moved. For this purpose, a brake or braking means 48 may be provided. When appropriate co-operating of engaging screw threads, as will be explained below, cause the hollow biopsy needle 11 to move forward and backward in an axial direction, the brake 48 prevents the inner needle 12 from rotating.

It may also cause the inner needle 12 to be prevented from moving in an axial direction when the hollow biopsy needle 11 is moved axially. The inner needle 12 may be provided as a metal hollow or solid inner needle, e.g. made of stainless steel or a non ferrite metal with high magnetic resistance. It is preferred that the cannula 10, hollow biopsy needle 11 and inner needle 12 as a whole are provided out of stainless steel or other rust-free metal, e.g. medical grade stainless steel, and/or a non ferrite metal or a material with high magnetic resistance.

Preferably, the cylindrical back end 17 and 26 of the cannula 10 and hollow biopsy needle 11 and the cylindrical body 34 of the inner needle 12 are made of a suitable plastic material of which polypropylene is only one example.

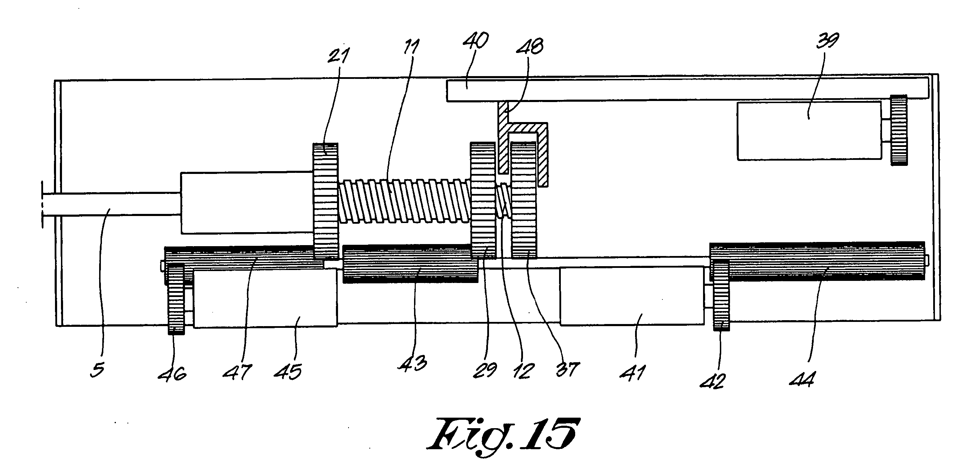

An explanation of how a pneumatic driving means 4 with biopsy needle assembly 5 is applied in order to have a tissue sample cut from an organ or other tissue, is now provided and reference is made to figures 15 to 18, showing the consecutive steps to be applied to sample a tissue.

The device comprises a first pneumatic motor 41, which drives a gear wheel 42, engaging with only the gear wheel 29 of the cylindrical back end 26 of the hollow biopsy needle 11 by means of a first counter gear wheel 43, or which may engage with the gear wheel 29 of the cylindrical back end 26 of the hollow biopsy needle 11 and the gear wheel 37 of the inner needle 12 by means of a second counter gear wheel 44.

The pneumatic driving means 4 comprises a second pneumatic motor 45, which drives the gear wheel 46 and the counter gear wheel 47, the latter engaging with the gear wheel 21 of the cylindrical back end 17 of cannula 10.

The pneumatic driving means 4 further comprises a third pneumatic motor 39, which by means of a set of gear wheels and a geared or timing belt 40, may move the hollow biopsy needle 11 and inner needle 12 forward and backward. The belt 40 is coupled to the gear wheel 37 of the inner needle 12 by means of a brake 48, which may prevent the gear wheel 37 from rotating when the hollow biopsy needle 11 is rotated by engaging with the rotating gear wheel 43.

Figure 15 shows the pneumatic driving means 4 in a starting position. The inner needle is screwed to its position where the cylindrical body 34 is closest to the cavity 22 of hollow biopsy needle 11. The cylindrical back end 26 of the hollow biopsy needle 11 is positioned in such a way that the screw thread 28 and screw thread 19 are engaged, but in a way that the only the engaging part of screwing thread 28 is located inside the cylindrical body end 17 of the cannula 10.

At the front end of the biopsy needle assembly, the cutting edge 15, the front end 23 of hollow biopsy needle 11 and the front end 32 of the inner needle 12 are substantially flush with each other or coterminous. Together they make a point to penetrate the tissues or organs up to the diseased site where a biopsy has to be taken. Preferably, the front end 32 of inner needle 12 is a sharp point, extending slightly out of the cutting edge 15 and front end 23 of hollow biopsy needle 11.

The biopsy needle assembly 5 is inserted in the tissue to be sampled, by penetrating the front end of the biopsy needle assembly 5 into the tissue, until the tissue to be sampled is flush with the front end of the biopsy needle assembly 5. Scanning equipment such as preferentially an MRI scanner, but also possibly a CT scanner, stereotactic radiology equipment or ultrasound scanner may be used to assist in locating the needle correctly.

A MRI scanner (Magnetic Resonance Imaging) is preferred to co-operate optimally with the biopsy device 1 according to the present invention, as no or limited magnetic disturbance is generated by such device.

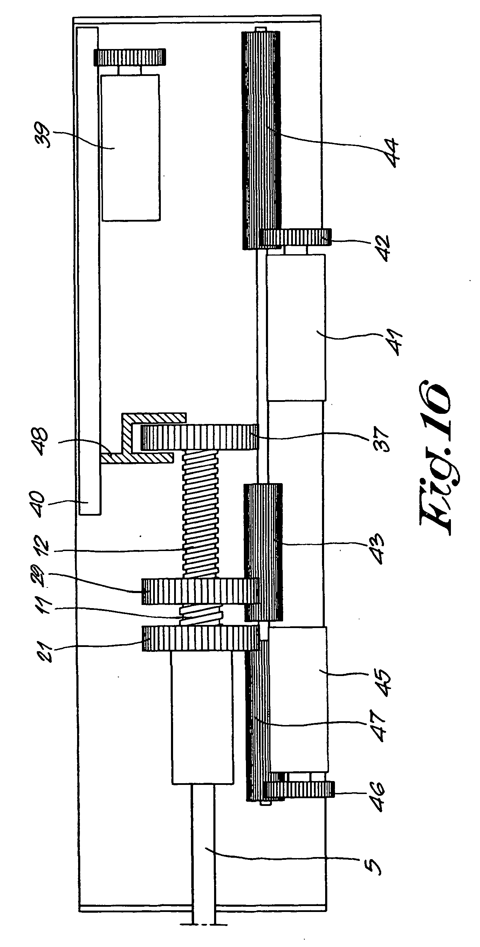

As shown in figure 16, the hollow biopsy needle 11 is only now brought forward. This is done by rotating the hollow biopsy needle 11 by means of engaging the gearwheel 29 with the rotating counter wheel 43, being driven by pneumatic motor 41. In the meantime, the brake 48 prevents the inner needle from rotating along with the hollow biopsy needle.

The engaging screw thread 35 of the inner needle and the screw thread 31 of the cylindrical back end 26 of the hollow biopsy needle 11 force the hollow biopsy needle 11 to move in the direction of the front end.

During this movement, the tissue-receiving element 25 is brought, by a screwing action, into the tissue to be sampled. Also during this rotation of the hollow biopsy needle 11, because the inner needle 12 is prevented rotating along with then hollow biopsy needle 11 by a braking force exerted by the braking means 48 on the inner needle 12, the inner needle 12 is prevented from moving axially with the hollow biopsy needle 11.

Thus, the inner space 38 is increased in volume and the front end 23 of the hollow biopsy needle 11 is axially moved away from the front end 32 of the inner needle 12, which is penetrating the tissue to be sampled.

Via the cavity 22, a vacuum, created in the inner space 38, is provided at the front end of the biopsy needle assembly, where the tissue to be sampled is sucked by the vacuum into the front end 32 of inner needle 12.

The cannula 10 is prevented from rotation along with the hollow biopsy needle 11, because the gear wheel 21 is prevented from rotation by engaging with the gear counter wheel 47 which is not driven at that moment but remains in a fixed position.

Because of the engagement of the screw thread 28 at the outer surface of the cylindrical back end 26 with the screw thread 19 at the inner side 18 of the cylindrical back end 17 of the cannula 10, the cylindrical back end 26 of the hollow biopsy needle 11 is actually screwed into the cylindrical back end 17 of the cannula 10.

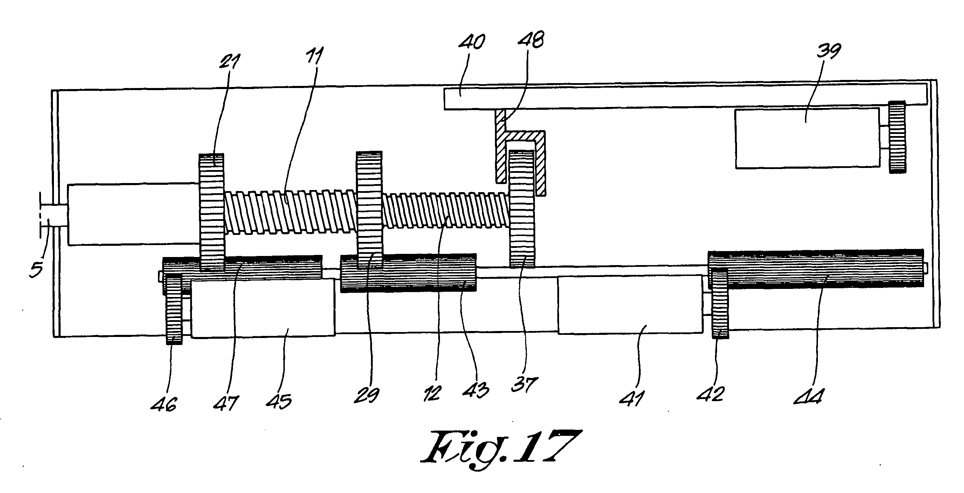

During the next step, as shown in figure 17, the hollow biopsy needle 11 and inner needle 12 are kept in a fixed position by having the gearwheel 29 of the hollow biopsy needle 11 in an engaged position with respect to the counter gear wheel 43, which is not driven.

The cannula 10 is rotated by means of the engagement of gear wheel 21 with counter gear wheel 47 is driven by a pneumatic motor 45.

Because of the engagement of the screwing thread 28 at the outer surface of the cylindrical back end 26 with the screw thread 19 at the inner side 18 of the cylindrical back end 17 of the cannula 10, the cannula 10 is axially moved forwards towards the front end of the tissue receiving means 25, meanwhile cutting the tissue sample which is screwed into the screwing shape of the tissue receiving means.

As the hollow biopsy needle 11 and inner needle 12 do not change position one with respect to the other, the vacuum which was created, is maintained and the sampled tissue is kept at the front end 32 of inner needle 12.

The cannula 10 is rotated until the screw thread 28 at the outer surface of the cylindrical back end 26 and the screw thread 19 at the inner side 18 of the cylindrical back end 17 of the cannula 10 disengage. The tissue sample is now present in the tissue receiving means 25.

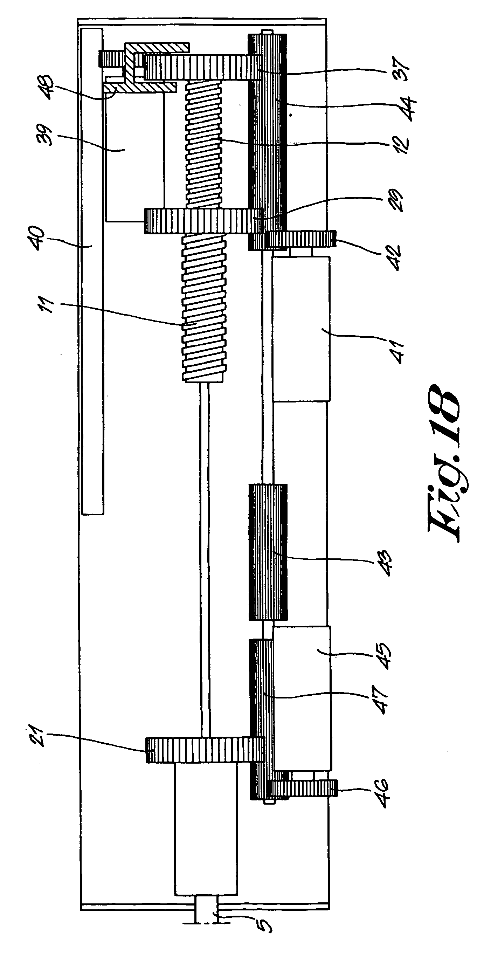

In a next step, as shown in figure 18, the inner needle 12 and hollow biopsy needle 11 are axially moved backwards by the means of a set of gear wheels and a timing or geared belt 40 over such a distance that the sampled tissue in the tissue receiving means is presented at the aperture 16 of the cannula 10, meanwhile bringing gear wheels 29 and 37 in engagement with counter gear wheel 44.

By rotation of the gear wheel 44, the inner needle 12 and hollow biopsy needle 11 is rotated simultaneously to line up with the aperture and the tissue can be removed from the receiving means 25.

During the removal of the tissue sample, the vacuum is interrupted by air, which is allowed to flow into the cavity and into the inner space 38.

In case another consecutive sample is to be taken, the cannula 10, hollow biopsy needle 11 and inner needle 12 are brought again into their starting position, as shown in figure 15, and the above steps may be repeated.

The cannula 10, hollow biopsy needle 11 and inner needle 12 are brought back again by a rotating co-axial movement of the inner needle 12 with the hollow biopsy needle 11 with respect to the cannula 10.

Once the cylindrical element of the hollow biopsy needle 11 meets the cylindrical element of the cannula 10, the cylindrical elements of both engage.

The system searches the reference position to repeat the biopsy procedure.

Possibly a marker element, such as a helix, loop or knot with the external diameter smaller than the internal diameter of the cutting cannula and with an expanding or tissue hooking effect in the target tissue, is provided on the tissue receiving means 25 brought at the height of the aperture or apertures 16.

By axially moving forward again of the hollow biopsy needle 11 and inner needle 12, the marker can be used to bring the biopsy needle at the position in the tissue, where the sample of the tissue was taken in previous steps.

As an example, in the biopsy needle assembly 5 as described by means of the figures 2 to 13, the following dimensions are used. The cannula 10 has an inner diameter D1 of about 3 to 4 mm.

The length of the biopsy needle assembly 5, which can be brought into the tissue to be sampled, this is the length L1 from the front end of the biopsy needle assembly 5 to the aperture 16, is about 100 mm.

The length L2 of the tissue receiving means 25, being preferably substantially equal with the length of the aperture or apertures 16 is about 18 to 20 mm. This length L2 is about the length over which the hollow biopsy needle 11 can move axially as compared to fixed cannula 10 and fixed inner needle 12. The length of the above mentioned co-operating screw treads is about L2. The axial displacement L3 that the cannula 10 is allowed to move is about 26 mm.

The length of axial displacement L4 of hollow biopsy needle 11 and inner needle 12, in order to provide the sampled tissue before the aperture 16 is about the maximal insertion length L1 of the biopsy needle assembly 5. In the embodiment as described, L4 is about 100 mm.

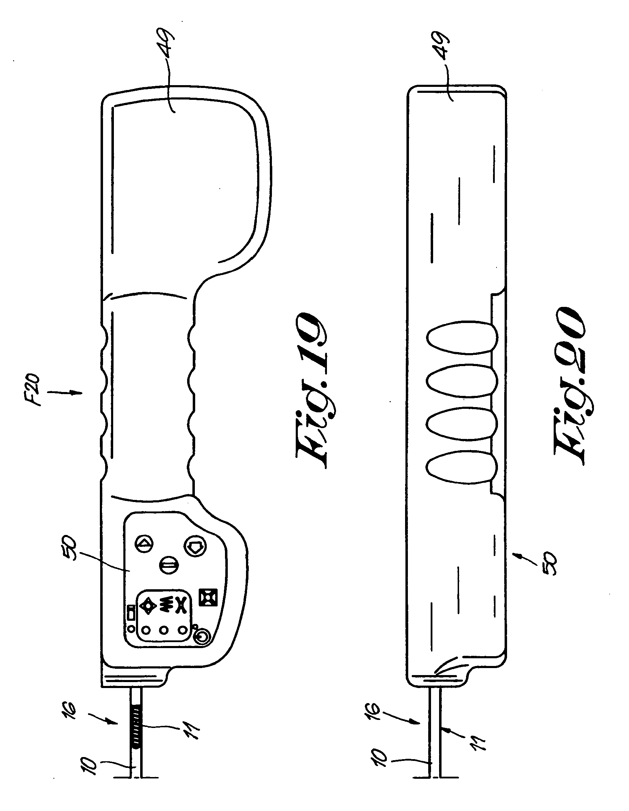

As shown in figure 19, in accordance with a further embodiment of the present invention, the pneumatic driving means 4 is provided with a hand held casing 49 which may comprise all the above mentioned elements, and which has a control panel 50 for controlling the manipulations of the device. The control panel has activating elements such as buttons for initiating and stopping the various manipulations described above, e.g. to start and stop the various pneumatic motors.

Although a device according to the present invention has taken all possible measures to avoid magnetic or electromagnetic interference with other instruments, according to a further embodiment of the device according to the present invention, the different pneumatic motors 41, 45, and 39 are placed in a separate housing and or in a magnetically shielded housing, eventually to be installed at distance or in a position where it cannot cause interference with devices carried by the operating physician or paramedics or with other equipment, medical or not.

The pneumatic motors are here coupled to the gear wheels 42, 43 and gearing to timing belt 40.

It is clear that the pneumatic driving means 4 can be organised in another way and can comprise other types of pneumatic components, such as linear displacement pistons.

It is also clear that the gas compressor 2 can be replaced by a hydraulic or liquid compressor, and that therefore the conducting means 3 are to be understood to embrace means for conducting compressed liquid.

The pneumatic driving means 4 are in such case replaced by hydraulic driving means.

The hydraulic system results in a more powerful device 1 according to the invention, and has the unexpected advantage of being even more precise and accurate in operation.

It is also clear that the gas or liquid compressor 2 can be completed or replaced by means of a gas or liquid aspirator providing a vacuum or a pressure below ambient pressure.

As such, between the compressor and/or aspirator 2 and needle assembly 5, a tube or a further tube can be added for providing a vacuum or low pressure to the needle assembly 5, so that the described piston-cylinder vacuum unit can be left away.

As a consequence, it is also clear that the transmission components, part of the driving means 4, can be different and adapted to the pneumatic driving components.

It is clear that the compressor and/or aspirator 2 can be replaced by any source of pressured gas or liquid, and as such should the word compressor 2 be interpreted.

The conducting means 3 can mainly exist of tubes or the like.

It is clear that the user interface 7 does not have to be provided in the same casing 49, and that it neither should be electrically connected with the pneumatic driving means 4, neither directly or via the steering unit 6.

The biopsy needle assembly 5 can be quite different from the embodiment as discussed above, and does not have to be mounted directly onto the pneumatic driving means 4.

The steering unit 6 does not have to be electronic, but the logic can be obtained by means of pneumatic, hydraulic or optic switches.

The steering unit 6 can be provided near the compressor 2, or anywhere else at a distance from the needle assembly 5, or even near the needle assembly 5 if appropriate logic components are used, for example pneumatic of optic, and/or if appropriate shielding is provided.

The steering unit 6 can also be provided in a distributed manner.

The present invention is by no means limited to the above described embodiments, on the contrary many alternatives of the device for taking a tissue sample according to the invention may be realised without departing from the scope of the invention.

## Claims

1. A device for taking a tissue sample, the device comprising a gas or liquid compressor and/or aspirator (2) and conducting means (3) for conducting compressed and/or vacuum or low-pressure gas or liquid, for example air, from the compressor and/or aspirator (2) to a biopsy needle assembly (5) comprising at least two components (10,11,12) which can be moved with respect to each other, which biopsy needle assembly (5) is in co-operation with a pneumatic driving means (4) which enables relative movement of at least one of the components (10,11,12) with respect to at least another component (10, 11, 12), wherein the components comprise a cannula (10), a hollow biopsy needle (11) and an inner needle (12), whereby the hollow biopsy needle (11) is provided with tissue receiving means (25) and can be located inside and coaxially with the cannula (10), and the inner needle (12) can be located inside and coaxially with the hollow biopsy needle (11), **characterised in that** the needle assembly (5) is provided with a piston-cylinder vacuum unit for creating a vacuum near the tissue receiving means (25) by axially moving two of said components of the needle assembly (5).

2. Device according to claim 1, **characterised in that** the needle assembly (5) comprises components which are made of materials which are magnetically passive.

3. Device according to claim 1, **characterised in that** the pneumatic driving means (4) comprises components which are made of materials which are magnetically passive and/or which are magnetically shielded.

4. Device according to claim 1, **characterised in that** the needle assembly (5) comprises a hollow piercing needle provided of a pointed distal end and thereafter a tissue receiving port, for example in the form of an aperture in the jacket of the needle; the needle assembly (5) further comprising an outer cannular cutter which fits precisely over the needle and is meant to be moved, eventually to be translated while eventually being rotated simultaneously, over said needle in order to cut of tissue which protrudes through the tissue receiving port.

5. Device according to claim 1, **characterised in that** the needle assembly (5) comprises a hollow piercing needle provided of a pointed distal end and thereafter a tissue receiving port, for example in the form of an aperture in the jacket of the needle; the needle assembly (5) further comprising an inner cannular cutter which fits precisely in the needle and is meant to be moved, eventually to be translated while eventually being rotated simultaneously, in said needle in order to cut of tissue which protrudes through the tissue receiving port.

6. Device according to any or more of the above claims, **characterised in that** the tissue receiving means (25), is a spirally shaped tissue receiving means (25), the outer free extremity of the spirally shaped tissue-receiving means preferably extending in a direction which is situated in the prolongation of the spiral course of the spirally shaped tissue-receiving means (25).

7. Device according to any one of the preceding claims, **characterised in that** the piston-cylinder vacuum unit, is situated in the prolongation of the needle assembly (5).

8. Device according to claim 1, **characterised in that** the pneumatic driving means (4) comprise at least one pneumatic motor (39,41,45).

9. Device according to claim 1, **characterised in that** the pneumatic driving means (4) comprise at least one linear displacement piston.

10. Device according to claim 1, **characterised in that** a steering unit (6) is provided, comprising electronic, optic, hydraulic and/or pneumatic logic components.

## Patentansprüche

1. Vorrichtung zur Entnahme einer Gewebeprobe, wobei die Vorrichtung einen Gas- oder Flüssigkeitsverdichter und/oder -sauger (2) umfasst und Leitungsmittel (3) zum Leiten von Druck- und/oder Vakuum- oder Niederdruckgas oder -flüssigkeit, beispielsweise Luft, von dem Verdichter und/oder Sauger (2) zu einer Biopsienadelanordnung (5), die mindestens zwei Komponenten (10, 11, 12), die in Bezug zueinander bewegt werden können, umfasst, welche Biopsienadelanordnung (5) mit einem pneumatischen Antriebsmittel (4) zusammenwirkt, das eine relative Bewegung mindestens einer der Komponenten (10, 11, 12) in Bezug auf mindestens eine andere Komponente (10, 11, 12) ermöglicht, wobei die Komponenten eine Kanüle (10), eine hohle Biopsienadel (11) und eine innere Nadel (12) umfassen, wobei die hohle Biopsienadel (11) mit Gewebeaufnahmemitteln (25) versehen ist und innerhalb und koaxial zu der Kanüle (10) angeordnet werden kann und die innere Nadel (12) innerhalb und koaxial zu der hohlen Biopsienadel (11) angeordnet werden kann, **dadurch gekennzeichnet, dass** die Nadelanordnung (5) mit einer Kolben-Zylinder-Vakuumeinheit zum Erzeugen eines Vakuums nächst dem Gewebeaufnahmemittel (25) durch axiales Bewegen von zwei der besagten Komponenten der Nadelanordnung (5) versehen ist.

2. Vorrichtung nach Anspruch 1, **dadurch gekennzeichnet, dass** die Nadelanordnung (5) Komponenten umfasst, die aus Materialien hergestellt sind, die magnetisch passiv sind.

3. Vorrichtung nach Anspruch 1, **dadurch gekennzeichnet, dass** das pneumatische Antriebsmittel (4) Komponenten umfasst, die aus Materialien hergestellt sind, die magnetisch passiv sind und/oder die magnetisch abgeschirmt sind.

4. Vorrichtung nach Anspruch 1, **dadurch gekennzeichnet, dass** die Nadelanordnung (5) eine hohle Durchstichnadel, die mit einem spitzen distalen Ende versehen ist, und danach eine Gewebeaufnahmeöffnung, beispielsweise in Form einer Öffnung im Mantel der Nadel, umfasst; wobei die Nadelanordnung (5) weiter einen äußeren Kanülen-Abschneider umfasst, der präzise über die Nadel passt und dazu bestimmt ist, über besagte Nadel bewegt zu werden, gegebenenfalls verschoben zu werden, während er gegebenenfalls gleichzeitig rotiert wird, um Gewebe abzuschneiden, das durch die Gewebeaufnahmeöffnung ragt.

5. Vorrichtung nach Anspruch 1, **dadurch gekennzeichnet, dass** die Nadelanordnung (5) eine hohle Durchstichnadel, die mit einem spitzen distalen Ende versehen ist, und danach eine Gewebeaufnahmeöffnung, beispielsweise in Form einer Öffnung im Mantel der Nadel, umfasst; wobei die Nadelanordnung (5) weiter einen äußeren Kanülen-Abschneider umfasst, der präzise in die Nadel passt und dazu bestimmt ist, in besagter Nadel bewegt zu werden, gegebenenfalls verschoben zu werden, während er gegebenenfalls gleichzeitig rotiert wird, um Gewebe abzuschneiden, das durch die Gewebeaufnahmeöffnung ragt.

6. Vorrichtung nach einem oder mehreren der vorgenannten Ansprüche, **dadurch gekennzeichnet, dass** das Gewebeaufnahmemittel (25) ein spiralförmiges Gewebeaufnahmemittel (25) ist, wobei das äußere freie Ende des spiralförmigen Gewebeaufnahmemittels sich bevorzugt in einer Richtung erstreckt, die sich in der Verlängerung des Spiralverlaufs des spiralförmigen Gewebeaufnahmemittels (25) befindet.

7. Vorrichtung nach gleich welchem der vorgenannten Ansprüche, **dadurch gekennzeichnet, dass** die Kolben-Zylinder-Vakuumeinheit sich in der Verlängerung der Nadelanordnung (5) befindet.

8. Vorrichtung nach Anspruch 1, **dadurch gekennzeichnet, dass** die pneumatischen Antriebsmittel (4) mindestens einen Pneumatikmotor (39, 41, 45) umfassen.

9. Vorrichtung nach Anspruch 1, **dadurch gekennzeichnet, dass** die pneumatischen Antriebsmittel (4) mindestens einen linearen Verdrängerkolben umfassen.

10. Vorrichtung nach Anspruch 1, **dadurch gekennzeichnet, dass** eine Steuerungseinheit (6) vorgesehen ist, die elektronische, optische, hydraulische und/oder pneumatische Logikkomponenten umfasst.

## Revendications

1. Dispositif pour prélever un échantillon de tissu, le dispositif comprenant un aspirateur et/ou un compresseur de gaz ou de liquide (2) et un moyen conducteur (3) pour amener le gaz ou le liquide comprimé et/ou sous vide ou sous basse pression, par exemple de l'air, depuis l'aspirateur et/ou le compresseur (2) jusqu'à un assemblage d'aiguille à ponction-biopsie (5) comprenant au moins deux composants (10, 11, 12) qui sont à même de se déplacer l'un par rapport à l'autre, ledit assemblage d'aiguille à ponction-biopsie (5) coopérant avec un moyen d'entraînement pneumatique (4) qui permet le mouvement relatif d'au moins un des composants (10, 11, 12) par rapport à au moins un autre composant (10, 11, 12), dans lequel les composants comprennent une canule (10), une aiguille creuse à ponction-biopsie (11) et une aiguille interne (12), l'aiguille creuse à ponction-biopsie (11) étant équipée d'un moyen de réception de tissu (25) et pouvant venir se disposer à l'intérieur de la canule (10) et en position coaxiale avec cette dernière, et l'aiguille interne (12) pouvant venir se situer à l'intérieur de l'aiguille creuse à ponction-biopsie (11) et en position coaxiale avec cette dernière, **caractérisé en ce que** l'assemblage d'aiguille (5) est muni d'une unité de vide à piston-cylindre pour créer un vide à proximité du moyen de réception de tissu (25) via le déplacement en direction axiale de deux desdits composants de l'assemblage d'aiguille (5).

2. Dispositif selon la revendication 1, **caractérisé en ce que** l'assemblage d'aiguille (5) comprend des composants qui sont constitués par des matériaux magnétiques passifs.

3. Dispositif selon la revendication 1, **caractérisé en ce que** le moyen d'entraînement pneumatique (4) comprend des composants qui sont constitués par des matériaux magnétiques passifs et/ou des matériaux munis d'un blindage antimagnétique.

4. Dispositif selon la revendication 1, **caractérisé en ce que** l'assemblage d'aiguille (5) comprend une aiguille de perçage creuse munie d'une extrémité distale pointue, à laquelle fait suite un orifice de réception de tissu, par exemple sous la forme d'une ouverture dans l'enveloppe de l'aiguille ; l'assemblage d'aiguille (5) comprenant en outre un instrument tranchant externe de forme tubulaire qui vient se disposer avec précision par-dessus l'aiguille et qui est destiné à se déplacer, en définitive à subir une translation tout en étant mis en définitive en rotation de manière simultanée, par-dessus ladite aiguille de façon à couper le tissu qui fait saillie à travers l'orifice de réception de tissu.

5. Dispositif selon la revendication 1, **caractérisé en ce que** l'assemblage d'aiguille (5) comprend une aiguille de perçage creuse munie d'une extrémité distale pointue, à laquelle fait suite un orifice de réception de tissu, par exemple sous la forme d'une ouverture dans l'enveloppe de l'aiguille ; l'assemblage d'aiguille (5) comprenant en outre un instrument tranchant interne de forme tubulaire qui vient se disposer avec précision dans l'aiguille et qui est destiné à se déplacer, en définitive à subir une translation tout en étant mis en définitive en rotation de manière simultanée, dans ladite aiguille de façon à couper le tissu qui fait saillie à travers l'orifice de réception de tissu.

6. Dispositif selon une ou plusieurs des revendications précédentes, **caractérisé en ce que** le moyen de réception de tissu (25) représente un moyen de réception de tissu en forme de spirale (25), l'extrémité libre externe du moyen de réception de tissu en forme de spirale s'étendant de préférence dans une direction qui se situe dans le prolongement de la spirale du moyen de réception de tissu (25) en forme de spirale.

7. Dispositif selon une ou plusieurs des revendications précédentes, **caractérisé en ce que** l'unité de vide à piston-cylindre est située dans le prolongement de l'assemblage d'aiguille (5).

8. Dispositif selon la revendication 1, **caractérisé en ce que** le moyen d'entraînement pneumatique (4) comprend au moins un moteur pneumatique (39, 41, 45).

9. Dispositif selon la revendication 1, **caractérisé en ce que** le moyen d'entraînement pneumatique (4) comprend au moins un piston à déplacement linéaire.

10. Dispositif selon la revendication 1, **caractérisé en ce qu'**on prévoit une unité de conduite (6) comprenant des composants logiques électroniques, optiques, hydrauliques et/ou pneumatiques.
